(19) Europäisches Patentamt / European Patent Office / Office européen des brevets

(11) EP 3 074 569 B1

(12) **FASCICULE DE BREVET EUROPEEN**

(45) Date de publication et mention de la délivrance du brevet:
**01.05.2019 Bulletin 2019/18**

(21) Numéro de dépôt: **14806579.0**

(22) Date de dépôt: **26.11.2014**

(51) Int Cl.:
***D21C 5/00*** (2006.01) ***C12P 7/00*** (2006.01)
***C12P 7/40*** (2006.01) ***C07C 65/00*** (2006.01)
***C07G 1/00*** (2011.01)

(86) Numéro de dépôt international:
**PCT/EP2014/075680**

(87) Numéro de publication internationale:
**WO 2015/078920 (04.06.2015 Gazette 2015/22)**

(54) **PROCÉDÉ DE DÉPOLYMÉRISATION DE LA LIGNINE PAR DES LACCASES**

VERFAHREN ZUR DEPOLYMERISIERUNG VON LIGNIN DURCH LACCASEN

PROCESS FOR DEPOLYMERIZATION OF LIGNIN BY LACCASES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.11.2013 FR 1361718**

(43) Date de publication de la demande:
**05.10.2016 Bulletin 2016/40**

(73) Titulaires:
- **Institut Polytechnique de Bordeaux**
  **33402 Talence Cedex (FR)**
- **Université de Bordeaux**
  **33000 Bordeaux (FR)**
- **Centre National de la Recherche Scientifique (C.N.R.S.)**
  **75016 Paris (FR)**

(72) Inventeurs:
- **GRELIER, Stéphane**
  **F-40160 Parentis en Born (FR)**
- **KOUMBA YOYA, Georges**
  **G1P 4B1 Québec city, QC (CA)**

(74) Mandataire: **Lavoix**
**2, place d'Estienne d'Orves**
**75441 Paris Cedex 09 (FR)**

(56) Documents cités:
**WO-A1-2013/090430**

- **SREBOTNIK E ET AL: "Degradation of nonphenolic lignin by the laccase/1-hydroxybenzotriazole system", JOURNAL OF BIOTECHNOLOGY, ELSEVIER SCIENCE PUBLISHERS, AMSTERDAM, NL, vol. 81, no. 2-3, 25 août 2000 (2000-08-25), pages 179-188, XP004210489, ISSN: 0168-1656, DOI: 10.1016/S0168-1656(00)00303-5**
- **BOURBONNAIS R ET AL: "Lignin oxidation by laccase isozymes from Trametes versicolor and role of the mediator 2,2'-azinobis(3-ethylbenzthiazoline-6-sulf onate) in kraft lignin depolymerization", APPLIED AND ENVIRONMENTAL MICROBIOLOGY, AMERICAN SOCIETY FOR MICROBIOLOGY, US, vol. 61, no. 5, 1 mai 1995 (1995-05-01), pages 1876-1880, XP002209033, ISSN: 0099-2240**
- **Patrick J Collins ET AL: "Reduction of the 2,2?-Azinobis(3-Ethylbenzthiazoline-6-Sulf onate) Cation Radical by Physiological Organic Acids in the Absence and Presence of Manganese", Copyright, 1 January 1998 (1998-01-01), pages 2026-2031, XP055479664, Retrieved from the Internet: URL:https://www.ncbi.nlm.nih.gov/pmc/artic les/PMC106274/pdf/am002026.pdf**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé de dépolymérisation de la lignine.

La lignine est le deuxième biopolymère renouvelable le plus abondant, après la cellulose, et, à elles deux, elles constituent plus de 70 % de la biomasse totale.

La valorisation de la lignine représente donc un enjeu important.

Dans l'industrie papetière, de grandes quantités de lignine sont obtenues à titre de sous-produits ou de déchets, dont seule une infime partie est valorisée sous forme chimique. Dans l'industrie sucrière, des quantités importantes de lignine sont également obtenues sous forme de déchets lors de l'extraction du suc de canne à sucre. La lignine est principalement utilisée en tant que liant ou dispersant, ou pour la préparation de biogaz par un traitement à haute température (800°C - 1000°C). Il existe également des méthodes de dépolymérisation de la lignine faisant intervenir des dérivés chlorés, non satisfaisantes d'un point de vue environnemental.

A l'heure actuelle, il existe peu de procédés viables économiquement utilisant la lignine comme matière première pour produire des composés chimiques.

Il existe donc un besoin pour un nouveau procédé de valorisation de la lignine, qui soit viable économiquement et acceptable d'un point de vue environnemental.

L'article "Dégradation of nonphenolic lignin by the laccase/1-hydroxybenzotriazole system", Srebotnik, E., et al., Journal of Biotechnology, vol. 81, no. 2-3, 25 Août 2000, pages 179-188, décrit un procédé pour la dépolymérisation de la lignine non-phénolique comprenant une étape d'oxydation de la lignine non-phénolique avec laccase en présence d'hydroxybenzotriazole dans un mélange comprenant de la N,N-diméthylformamide et en présence d'air. Le document WO 2013/090430 décrit un procédé impliquant la dégradation de la lignine non-phénolique comprenant la mise en contact de la lignine avec une lignine dépolymérase (laccase) et un composé auxiliaire. L'article "Lignin oxidation by laccase isozymes from Trametes versicolor and role of the mediator 2,2'-azinobis(3-ethylbenzthiazoline-6-sulfonate) in kraft lignin depolymerization", Bourbonnais, R., et al., Applied and Environmental Microbiology, vol. 61, no. 5, 1 Mai 1995, pages 1876-1880, décrit un procédé pour la dépolymérisation de la lignine non-phénolique comprenant une étape d'oxydation de la lignine avec un système comprenant laccase et 2,2-azino-bis-(3-éthylbenzothiazoline-6-acide sulfonique) (ABTS). L' article "Involvement of lipid peroxidation in the dégradation of a non-phenolic lignin model compound by manganese peroxidase of the litter-decomposing fungus Stropharia coronilla", Kapich, A., et al., Biochemical and Biophysical Research Communications, vol. 330, no. 2, 6 Mai 2005, pages 371-377, décrit un procédé de dégradation d'un composé modèle de lignine non-phénolique par la peroxydase de manganèse. L'article "Réduction of the 2,2'-Azinobis(3-Ethylbenzthiazoline-6-Sulfonate) Cation Radical by Physiological Organic Acids in the Absence and Presence of Manganese", Patrick J Collins, Alan D W Dobson, Jim A Field, Applied and Environmental Microbiology, Vol. 64, No. 6, Juin 1998, p. 2026-2031, décrit un procédé pour le recyclage de radicaux cationiques de ABTS sous leur forme réduite, de sorte qu'ils peuvent être réutilisés. Le document décrit que la concentration de médiateur redox utilisée dans les traitements des composés non phénoliques tels que la pulpe-lignine avec de laccase peut être réduite.

La présente invention a pour but de proposer un nouveau procédé de dépolymérisation de la lignine qui soit efficace et adapté d'un point de vue environnemental, permettant d'accéder à des produits de dépolymérisation valorisables dans différents domaines industriels, tels que les bioraffineries, l'agroalimentaire ou encore les cosmétiques.

Un autre but de la présente invention est de proposer un procédé permettant d'accéder, de manière contrôlée, à des produits de dépolymérisation de tailles diverses comme l'acide 3,4-diméthoxybenzoïque (acide vanillique méthylé), le vératraldéhyde (vanilline méthylée), le vératrole (gaïacol méthylé), ou le diformyl gaïacol méthylé.

Le procédé de la présente invention propose de combiner, de manière séquentielle, l'action d'une enzyme et l'action d'un agent oxydant.

Plus particulièrement, la présente invention concerne un procédé de dépolymérisation de la lignine comprenant :

- une étape d'oxydation de lignine non-phénolique par mise en présence, dans au moins un solvant, de lignine non-phénolique, de laccase, d'un médiateur rédox choisi dans le groupe constitué du 2,2-azino-bis-(3-éthylbenzothiazoline-6-acide sulfonique) (ABTS), de l'acide violurique, de l'acide hydroxyanthranilique, du TEMPO et du N-hydroxyphtelimide,et d'une source d'oxygène, ce par quoi on obtient un mélange comprenant de la lignine non-phénolique oxydée, et
- une étape de dépolymérisation de la lignine non-phénolique oxydée ainsi obtenue, par addition d'un agent oxydant nucléophile,

ladite lignine non-phénolique étant de la lignine alkylée obtenue à partir de lignine phénolique par fonctionnalisation de fonctions phénol de ladite lignine phénolique par des alkyles, pour donner des fonctions alcoxy.

Le procédé de l'invention est non-microbiologique, au sens où il n'utilise pas de microorganismes (tels que des champignons) exprimant de manière endogène des laccases.

Les inventeurs ont observé, de manière surprenante, que l'action sur de la lignine non-phénolique d'une laccase

en présence d'un médiateur rédox et d'une source d'oxygène, suivie de l'addition d'un agent oxydant, a pour effet de dépolymériser efficacement la structure de la lignine.

Lors de la première étape du procédé (étape d'oxydation), les fonctions hydroxyle en position benzylique de la lignine non-phénolique sont oxydées en fonctions cétone. Lors de cette étape, la structure polymérique de la lignine n'est pas fragmentée et sensiblement aucune liaison C-C n'est coupée.

Lors de la deuxième étape du procédé (étape de dépolymérisation, aussi appelée fragmentation), des liaisons C-C de la structure polymérique de la lignine sont rompues. Sans vouloir être lié à une théorie particulière, les liaisons C-C ainsi rompues sont celles qui se trouvent au voisinage des fonctions cétone formées lors de la première étape du procédé.

Le procédé de l'invention peut être représenté par le schéma illustratif suivant :

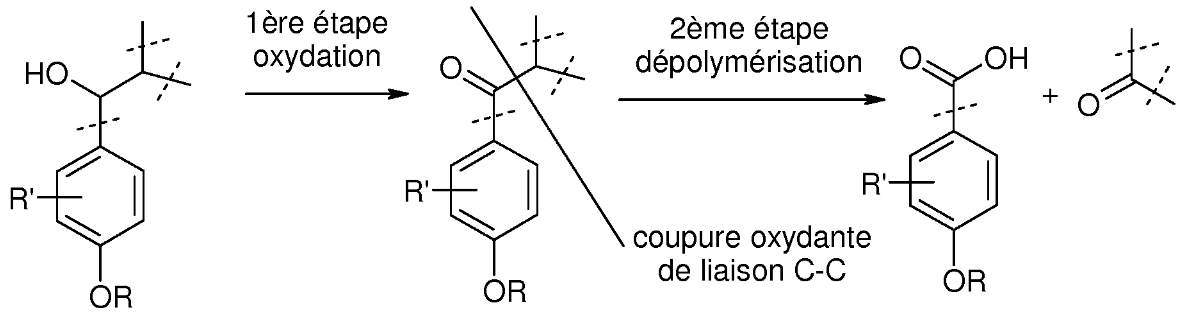


dans lequel le groupe -OR représente une fonction phénol fonctionnalisée de la lignine non-phénolique et le groupe -R' représente un éventuel autre substituant (différent d'une fonction phénol) du groupe phényle représenté. La présence, le nombre et le type de groupe -R' dépendent de la lignine utilisée. Il peut par exemple s'agir d'un ou plusieurs groupes méthoxy.

Le schéma ci-dessus est purement illustratif et ne reflète que schématiquement la structure de la lignine.

Comme cela sera exposé plus loin, les deux étapes du procédé s'effectuent avantageusement à la suite l'une de l'autre, dans le même réacteur.

Les conditions réactionnelles des étapes du procédé de l'invention seront décrites plus loin.

De manière générale, dans le cadre de la présente demande, on entend par « milieu réactionnel » le milieu dans lequel ont lieu les étapes du procédé et qui comprend un dérivé de lignine, une laccase, un médiateur rédox, une source d'oxygène, au moins un solvant, et éventuellement un agent oxydant (selon s'il a déjà été ajouté ou non).

Par « mise en présence », on entend ainsi l'addition d'un réactif dans le milieu réactionnel.

Ledit milieu réactionnel peut également comprendre d'autres réactifs tels qu'ils sont décrits dans la présente demande.

La matière première mise en oeuvre dans le procédé, à savoir la lignine non-phénolique, va maintenant être décrite.

Lignine non-phénolique

Par « lignine non-phénolique », on entend un dérivé de lignine phénolique, qui est le produit de réaction de la fonctionnalisation de fonctions phénol (Ph-OH) d'une lignine phénolique (aussi simplement appelée « lignine »).

Une lignine non-phénolique est une lignine modifiée ou fonctionnalisée, dans laquelle au moins une fonction phénol est modifiée. De préférence, au moins 50%, voire au moins 60%, voire au moins 70%, voire au moins 80%, voire au moins 90%, voire au moins 95%, voire au moins 99% des fonctions phénol de la lignine sont modifiées.

Un type de lignine non-phénolique est notamment décrite dans Srebotnik et al. (Journal of Biotechnology 81 (2000), 179-188).

De préférence, la lignine non-phénolique ne comporte pas de fonction phénol libre (non fonctionnalisée).

Une lignine non-phénolique est obtenue par réaction d'une lignine phénolique avec un agent de fonctionnalisation apte à convertir des fonctions phénol de ladite lignine phénolique en fonctions non réactives lors de l'étape d'oxydation du procédé. La fonctionnalisation de la lignine a pour but de protéger les fonctions phénol lors de l'étape d'oxydation du procédé et de les empêcher de réagir. Les fonctions phénol fonctionnalisées de la lignine non-phénolique ne sont ainsi pas réactives lors de l'étape d'oxydation par les laccases.

Dans le cadre de la présente invention, on entend par « fonctionnalisation de la lignine » la fonctionnalisation sélective des fonctions phénol, c'est-à-dire que les autres fonctions hydroxyle de la lignine (alcools aliphatiques en particulier) ne sont pas fonctionnalisées. De préférence, la fonctionnalisation est totale, c'est-à-dire que toutes les fonctions phénol sont fonctionnalisées.

Les inventeurs ont découvert qu'en utilisant une lignine phénolique conformément au procédé de l'invention, on améliore la dépolymérisation de la lignine.

L'alkylation est un exemple de fonctionnalisation convenant à la mise en oeuvre du procédé. On utilise pour cela un agent d'alkylation à titre d'agent de fonctionnalisation.

Selon un mode de réalisation, on peut utiliser de la lignine alkylée à titre de lignine non-phénolique.

Par « lignine alkylée », on entend de la lignine non-phénolique dans laquelle les fonctions phénol sont fonctionnalisées par des alkyles (typiquement en $C_1$-$C_{12}$), pour donner des fonctions alcoxy.

De préférence, la lignine alkylée ne comporte pas de fonction phénol libre, c'est-à-dire qu'elles sont toutes (ou quasiment toutes) sous forme de fonctions alcoxy.

Selon un mode de réalisation avantageux, on peut utiliser de la lignine méthylée à titre de lignine non-phénolique.

On utilise pour cela un agent de méthylation à titre d'agent de fonctionnalisation.

Par « lignine méthylée », on entend de la lignine alkylée dans laquelle les fonctions phénol sont fonctionnalisées par des méthyles, pour donner des fonctions méthoxy.

De préférence, la lignine méthylée ne comporte pas de fonction phénol libre, c'est-à-dire qu'elles sont toutes (ou quasiment toutes) sous forme de fonctions méthoxy.

Après la fonctionnalisation d'une lignine phénolique, afin de contrôler le taux de fonctionnalisation et la sélectivité de fonctionnalisation, on peut quantifier par RMN $^{31}P$ la quantité de fonctions phénoliques éventuellement restantes (qui n'auraient donc pas été fonctionnalisées). Une méthode est notamment décrite dans Granat et al. J. Agric. Food Chem., 1995, 43(6), 1538-1544.

Selon un mode de réalisation, la lignine non-phénolique est une lignine alkylée qui est obtenue par mise en présence, dans une solution aqueuse basique, de lignine phénolique et d'un agent alkylant.

Dans le cadre de la présente invention, un agent alkylant est un composé apte à fonctionnaliser les fonctions phénol par des groupes alkyle, en substituant les atomes d'hydrogène desdites fonctions phénol par des groupes alkyle (pour donner des fonctions alcoxy).

De préférence, on utilise un agent alkylant permettant d'alkyler sélectivement les fonctions phénol de la lignine, c'est-à-dire que les autres fonctions hydroxyle de la lignine (alcools secondaires en particulier) ne sont pas alkylées en fonctions alcoxy.

De préférence, on utilise un agent alkylant permettant d'alkyler totalement fonctions phénol de la lignine, c'est-à-dire que toutes (ou quasiment toutes) les fonctions phénol de la lignine sont alkylées en fonctions alcoxy.

Selon un mode de réalisation avantageux, la lignine non-phénolique est une lignine méthylée, qui est de préférence obtenue par mise en présence, dans une solution aqueuse basique, de lignine et d'un agent méthylant.

Dans le cadre de la présente invention, un agent méthylant est un composé apte à fonctionnaliser les fonctions phénol par des groupes méthyle, en substituant les atomes d'hydrogène desdites fonctions phénol par des groupes méthyle.

De préférence, on utilise un agent méthylant permettant de méthyler sélectivement les fonctions phénol de la lignine, c'est-à-dire que les autres fonctions hydroxyle de la lignine (alcools secondaires en particulier) ne sont pas méthylées en fonctions méthoxy.

La mise en présence de l'agent méthylant est typiquement effectuée au goutte-à-goutte.

La mise en présence est typiquement suivie d'une étape de chauffage du milieu réactionnel, à une température comprise typiquement de 50°C à 100°C.

A titre d'agent méthylant, on peut citer le diméthylsulfate (ou sulfate de diméthyle), le carbonate de diméthyle (DMC), l'iodure de méthyle et le diazométhane.

De préférence, on utilise le diméthylsulfate, qui permet de méthyler totalement et sélectivement les fonctions phénol d'une lignine phénolique.

L'étape de méthylation de la lignine peut être typiquement effectuée selon une méthode décrite dans Sadeghifar et al. Ind. Eng. Chem. Res. 2012, 51, 16713-16720.

La lignine est dissoute dans une solution aqueuse basique, typiquement de soude (par exemple à 0,7 M). Le mélange obtenu est agité, typiquement à température ambiante, et un agent méthylant est additionné, de préférence au goutte à goutte (à raison d'environ 50 équivalents d'agent méthylant par rapport au nombre d'équivalents de fonctions phénol). Le mélange est typiquement chauffé (par exemple à 80°C) jusqu'à ce que les réactifs soient consommés. Le mélange est alors acidifié, filtré et le solide obtenu est lavé à l'eau distillée et séché pour fournir la lignine méthylée.

Sans vouloir être lié à une théorie particulière, les inventeurs ont observé que la méthylation de la lignine, en bloquant sélectivement et totalement les fonctions phénols, permet d'éviter de manière très efficace les réactions de polymérisation de la lignine lors de l'action de la laccase, et ainsi d'améliorer la dépolymérisation.

L'effet de la méthylation est notamment illustré dans les exemples comparatifs décrits ci-après. Une lignine non-phénolique (non méthylée) traitée par le procédé de l'invention n'est pas dépolymérisée.

Lignine phénolique

Dans le cadre de la présente invention, on entend par « lignine phénolique » ou « lignine », une forme naturelle de lignine, comportant des fonctions phénol libres (Ph-OH).

La structure de la lignine se présente sous la forme d'un réseau tridimensionnel complexe, issu de la polymérisation d'unités de base qui ont pour squelette un motif phénylpropane. La lignine désigne donc plus généralement « des » lignines, en fonction des unités de base qui la constitue. Parmi les unités de base de la lignine, aussi appelées monolignols, on peut citer principalement l'alcool paracoumarylique, l'alcool coniférylique et l'alcool sinapylique.

Dans le cadre la présente invention, on entend par « dépolymérisation » une réaction au cours de laquelle des liaisons covalentes de la structure polymérique de la lignine sont rompues, conduisant à des produits de dépolymérisation de taille inférieure à la lignine de départ.

Par « taille inférieure », on entend que les produits de dépolymérisation obtenus selon le procédé de l'invention possèdent une masse moléculaire inférieure à celle de la lignine non-phénolique de départ.

Toutefois, dans le cadre la présente invention, le sens du terme « dépolymérisation » ne doit pas être limité à la transformation de la lignine non-phénolique en unités de base telles que décrites ci-dessus.

Typiquement, les produits de dépolymérisation de la lignine obtenus selon le procédé de l'invention sont formés d'une unité de base, de quelques unités de base, voire de quelques dizaines d'unités de base. On obtient généralement des mélanges de produits de dépolymérisation, comportant un nombre différent d'unités de base.

Généralement, étant donné la structure complexe de la lignine, le procédé de l'invention fournit un mélange de produits de dépolymérisation de tailles et de structures diverses, qui peuvent porter différents groupes fonctionnels, tels que des groupes cétone, aldéhyde, acide, phénol, alcool ou méthoxy par exemple, de préférence acide, méthoxy, alcool et cétone.

Lesdits produits de dépolymérisation peuvent être définis comme des « monomères », des « oligomères » ou des « fragments » de lignine non-phénolique.

Ainsi, par « procédé de dépolymérisation », on entend également un « procédé de fragmentation » de la structure de la lignine.

Les produits de dépolymérisation obtenus selon le procédé de l'invention présentent une taille comprise entre 100 g/mol et 5 000 g/mol, de préférence entre 150 g/mol et 4 000 g/mol, avantageusement entre 200 g/mol de 3 000 g/mol, préférentiellement entre 500 g/mol et 2 500 g/mol.

Le mélange de produits de dépolymérisation de la lignine est typiquement caractérisé par sa masse moléculaire moyenne $M_w$, qui représente la moyenne des masses molaires pondérée par la masse de chaque produit, et qui a pour formule :

$$M_w = \frac{\sum_i n_i M_i^2}{\sum_i n_i M_i}$$

dans laquelle $n_i$ désigne le nombre de produits i et $M_i$ désigne la masse moléculaire du produit i.

Pour un mélange de produits de dépolymérisation obtenu selon le procédé de l'invention, la masse moléculaire moyenne, $M_w$, peut être mesurée par chromatographie d'exclusion stérique (SEC pour « Size Exclusion Chromatography »).

Le mélange de produits de dépolymérisation présente typiquement une masse moléculaire moyenne $M_w$ comprise entre 100 g/mol et 5 000 g/mol, de préférence entre 150 g/mol et 4 000 g/mol, avantageusement entre 200 g/mol de 3 000 g/mol, préférentiellement entre 500 g/mol et 2 500 g/mol.

Le mélange de produits de dépolymérisation obtenu selon le procédé de l'invention peut également être caractérisé par le rapport molaire entre la masse moléculaire moyenne en masse $M_w$ dudit mélange et la masse moléculaire moyenne en masse de la lignine de départ $M_{lig}$.

Typiquement, en utilisant le procédé de l'invention, on obtient un rapport molaire $M_w/M_{lig}$ compris entre 1/3 et 1/10.

La lignine non-phénolique servant de matière première dans le procédé de l'invention peut être obtenue par toute méthode de fonctionnalisation (alkylation, méthylation, ...) connue en soi, à partir de toute source de lignine phénolique disponible, qu'elle soit commerciale ou bien extraite de résidus industriels riches en lignine.

A titre de source de lignine phénolique, on peut utiliser les lignines décrites ci-dessous, et de préférence la lignine Kraft (par exemple issue de la liqueur noire), éventuellement purifiée, ou bien la lignine de bagasse de canne à sucre, ou encore toute autre lignine industrielle.

Les lignines utilisables dans le cadre de la présente invention sont notamment décrites dans Lignins and

Lignans : Advances in Chemistry, Cyril Heitne, John Schmidt, CRC Press, Taylor&Francis Group, 2010.

Avantageusement, la lignine a subi un prétraitement de manière à la rendre soluble en milieu aqueux.

A titre de lignine phénolique, on peut utiliser de la lignine Kraft, par exemple extraite de liqueur noire.

Les lignines Kraft (aussi appelées thiolignines) sont des composés hydrosolubles issus de la production industrielle de la pâte à papier par utilisation d'ions sulfates.

La lignine Kraft utilisable dans le procédé de l'invention est typiquement obtenue par extraction de liqueur noire, qui est la liqueur de cuisson issue de la fabrication du papier Kraft. Il s'agit d'une solution aqueuse composée des résidus de lignine et d'hémicellulose dissous de la pâte à papier, ainsi que d'autres composés chimiques inorganiques (sels dissous).

La purification de la liqueur noire est typiquement effectuée comme suit. On acidifie la liqueur noire pour faire précipiter la lignine. Le solide obtenu est centrifugé, isolé et extrait à l'éthanol. Les fractions liquides sont récupérées et concentrées en évaporant l'éthanol. Le résidu est lavé par une solution acide pour éliminer les sels résiduels. On obtient ainsi de la lignine Kraft.

La lignine Kraft extraite de liqueur noire peut être en outre purifiée comme suit pour éliminer les impuretés (typiquement des composés monophénols et des résidus de dégradation). On met en présence la lignine Kraft avec du tétrahydrofurane, on filtre le mélange obtenu pour éliminer le solide, on concentre le filtrat en évaporant le solvant, puis on lave le solide obtenu avec du diéthyléther pour éliminer des impuretés organiques, tels que des composés monophénols (vanilline, acide vanillique, ...) et des résidus de dégradation. Le solide récupéré est séché pour donner de la lignine Kraft purifiée.

A titre de lignine phénolique, on peut également utiliser de la lignine issue de la bagasse de canne à sucre.

La bagasse de canne à sucre est le résidu fibreux de la canne à sucre obtenu après extraction du suc. Elle constitue un déchet important de l'industrie sucrière, qui n'est pas assez valorisé. La lignine de bagasse de canne à sucre est disponible commercialement (Solvay).

On peut aussi utiliser une source de lignine industrielle ou bien des mélanges de composés comprenant des lignines, typiquement des mélanges de biomasse comprenant de la lignine et d'autres constituants, tels que de la cellulose et/ou de l'hémicellulose, ou bien encore un mélange de lignines Kraft.

La lignine utilisée dans le procédé de l'invention présente typiquement une masse moléculaire moyenne en masse ($M_{lig}$) comprise entre 1 000 et 10 000 g/mol, de préférence entre 1 000 et 5 000 g/mol.

La masse moléculaire moyenne en masse de la lignine de départ ($M_{lig}$) représente la moyenne des masses molaires pondérée par la masse de chaque taille de lignine, et a pour formule :

$$M_{lig} = \frac{\sum_j n_j M_j^2}{\sum_j n_j M_j}$$

dans laquelle $n_j$ désigne le nombre de molécules de lignine de taille j et $M_j$ désigne la masse moléculaire d'une lignine de taille j.

Cette valeur est généralement indiquée par le fournisseur de lignine ou bien peut être déterminée par une mesure de chromatographie d'exclusion stérique, comme décrit plus haut.

Les étapes du procédé de l'invention vont maintenant être décrites.

Etape d'oxydation par les laccases

La première étape du procédé de l'invention consiste à mettre en présence, dans au moins un solvant, de la lignine non-phénolique telle que décrite ci-dessus, une laccase, un médiateur rédox et une source d'oxygène.

Cette étape conduit à l'oxydation de la lignine non-phénolique par l'action du système laccase/médiateur en présence d'une source d'oxygène, ce par quoi on obtient un mélange comprenant de la lignine non-phénolique oxydée.

Par « lignine non-phénolique oxydée », on entend une lignine non-phénolique telle que définie ci-dessus dans laquelle les fonctions hydroxyle en position benzylique ont été converties sélectivement en des fonctions cétone.

Lors de cette étape, la lignine non-phénolique n'est essentiellement pas dépolymérisée, c'est-à-dire que cette étape n'affecte pas (ou très peu) la structure polymérique de la lignine, en particulier elle ne rompt pas (ou très peu) de liaisons C-C.

Laccase

Les laccases (EC 1.10.3.2) sont une famille d'enzymes que l'on retrouve dans de nombreuses plantes, champignons et micro-organismes.

*In vivo,* les laccases ont une activité oxydante et agissent comme catalyseur au sein d'un processus d'oxydation enzymatique.

Les laccases utilisables dans le procédé de l'invention peuvent être issues de plantes, de champignons ou de micro-organismes. Les laccases issues de champignons incluent notamment les laccases des genres *Aspergillus, Neurospora* (par exemple *Crassa Neurospora), Podospora, Botrytis, Collybia, Fomes, Lentinus, Pleurotus, Trametes* (par exemple *Trametes villosa* et *Trametes versicolor*)*, Rhizoctonia* (par exemple *Rhizoctonia solani*)*, Coprinus* (par exemple *Coprinus cinereus, Coprinus comatus, Coprinus friesii* et *Coprinus plicatilis*)*, Psathyrella* (par exemple *Psathyrella condelleana*)*, Panaeolus* (par exemple *Panaeolus papilionaceus*)*, Myceliophthora* (par exemple *Myceliophthora thermophila*)*, Schytalidium* (par exemple *Schytalidium thermophilum*)*, Polyporus* (par exemple *Polyporus pinsitus), Phlebia* (par exemple *Radiata phlebia*)*, Pycnoporus* (par exemple *Pycnoporus cinnabarinus)* ou *Coriolus* (par exemple *Coriolus hirsutus*). Les laccases issues de bactéries sont par exemple issues de *Bacillus.*

De préférence, on utilise une laccase issue de *Trametes versicolor,* commercialisée par Sigma Aldrich.

Le rapport de la quantité de laccase mise en présence (en mg) sur la quantité initiale de lignine non-phénolique présente dans le milieu réactionnel (en grammes) est généralement compris entre 0,1/1 et 5/1.

Dans le cadre de leur utilisation *in vitro,* pour l'oxydation d'un substrat constitué de molécules volumineuses (typiquement de masse moléculaire supérieure à 5 000 g/mol), les laccases sont généralement associées à un « médiateur rédox », qui est un composé chimique de petite taille (typiquement de masse moléculaire inférieure à 1 000 g/mol) agissant comme un intermédiaire rédox entre les molécules de laccases et les molécules de substrat à oxyder. Les médiateurs rédox sont notamment décrits dans Bourbonnais R. Appl. Enviro. Microbiol. 1995, 61, 1876-1880.

Les médiateurs rédox sont également désignés comme des agents de transfert d'électrons car ils facilitent le transfert électronique entre les laccases et le substrat à oxyder.

Le principe de la médiation rédox est une technologie connue en soi.

Dans le cadre du traitement de la lignine par des laccases, en l'absence d'un médiateur rédox, les interactions chimiques entre les laccases, qui sont des protéines de grande taille, et les molécules de lignine, qui sont des polymères de grande taille, seraient défavorisées. On ajoute ainsi un médiateur rédox afin d'accélérer le processus d'oxydation enzymatique catalysé par les laccases.

Le rapport de la quantité de médiateur rédox mis en présence (en mmol) sur la quantité de laccase mise en présence (en mg) est généralement compris entre 1/1 et 1/50.

De préférence, le médiateur rédox mis en oeuvre selon le procédé de l'invention est le 2,2-azino-bis-(3-éthylbenzothiazoline-6-acide sulfonique) (ABTS).

Ce médiateur est particulièrement adapté à l'oxydation de lignine par les laccases.

Contrairement à d'autres médiateurs rédox, tels que l'hydroxybenzotriazole (HOBt) qui se dégrade rapidement en présence de laccase en perdant son activité de médiateur (HOBt devient HBt par perte d'atome d'oxygène), l'ABTS présente une meilleure activité en tant que médiateur rédox.

Les médiateurs rédox suivants peuvent également convenir à la mise en oeuvre de l'invention : acide violurique, acide hydroxyanthranilique, le TEMPO, le N-hydroxyphtelimide (Chakar et al. Can. J. Chem. 82: 344-352 (2004)).

## Source d'oxygène

Par « source d'oxygène », on entend un réactif apte à régénérer (réoxyder) les sites actifs de la laccase intervenant dans le cadre du processus d'oxydation enzymatique mentionné ci-dessus, qui fournit *in fine* de la lignine non-phénolique oxydée à partir de la lignine non-phénolique. Par « oxygène », on entend ici le dioxygène ($O_2$).

De préférence, la source d'oxygène est un gaz comprenant de l'oxygène, tel l'air ou l'oxygène pur.

A titre de source d'oxygène, on peut citer l'oxygène pur ($O_2$), que l'on met en présence, par bullage à pression atmosphérique ou sous une pression de quelques bars, dans le milieu réactionnel comprenant, au début de l'étape d'oxydation, de la lignine non-phénolique, une laccase, un médiateur rédox et au moins un solvant. On parvient ainsi à saturer avantageusement le milieu réactionnel en oxygène dissout.

A titre de source d'oxygène, on peut également citer l'air ou tout mélange de gaz enrichi en oxygène.

Par « mise en présence d'une source d'oxygène », on entend l'introduction de ladite source d'oxygène dans le milieu réactionnel du procédé de l'invention. Ladite mise en présence peut être effectuée de manière ponctuelle ou prolongée, de préférence prolongée, l'objectif recherché étant de saturer le milieu réactionnel en oxygène dissout.

Alternativement, on peut utiliser, à la place de la source d'oxygène, tout oxydant apte à régénérer (réoxyder) les sites actifs de la laccase intervenant dans le processus oxydatif enzymatique décrit ci-dessus.

La première étape du procédé est avantageusement effectuée dans des conditions de pH et de température appropriées à la réactivité de la laccase utilisée, c'est-à-dire dans des conditions de pH et de température qui ne dénaturent pas les propriétés de la laccase.

De préférence, la première étape du procédé de l'invention est effectuée en milieu acide, typiquement dans

un tampon à pH = 4.

De préférence, la première étape du procédé de l'invention est effectuée à une température comprise entre 20°C et 60°C, de préférence autour de 40°C.

Ces conditions sont particulièrement adaptées à la mise en oeuvre de la laccase issue de *Trametes versicolor*.

On pourra adopter les conditions de pH et de température optimales associées au type de laccase utilisé, ces conditions étant généralement connues pour une laccase donnée.

La première étape du procédé selon l'invention est effectuée en présence d'au moins un solvant.

Selon un mode de réalisation, le solvant est un mélange comprenant de l'eau et un solvant organique polaire.

De préférence, le solvant est un mélange comprenant une solution tampon de pH acide (typiquement à pH = 4) et un solvant organique polaire.

L'utilisation d'un tel mélange à titre de solvant a pour avantage de solubiliser la lignine non-phénolique ainsi que l'ensemble des réactifs mis en présence. On obtient ainsi un mélange réactionnel homogène, dans lequel les interactions chimiques sont facilitées.

Selon ce mode de réalisation, le solvant comprend de préférence entre 30% et 70% en volume d'eau, avantageusement entre 40% et 60%, par rapport au volume total de solvant.

Le complément de solvant est typiquement constitué d'un solvant organique polaire, de préférence suffisamment volatile pour être séparé des produits de dépolymérisation par évaporation, éventuellement sous pression réduite.

Le solvant est typiquement un mélange d'eau pour solubiliser la laccase et d'un éther apte à solubiliser la lignine non-phénolique, comme le dioxane. Un mélange dioxane/eau (1/1) peut typiquement être utilisé.

La première étape est effectuée selon une dilution telle que pour 1 g de lignine non-phénolique, on utilise un volume de solvant compris de 10 mL à 100 mL, de préférence de 30 mL à 70 mL, typiquement de 50 mL.

L'homme du métier sera en mesure d'adapter la dilution du milieu réactionnel en fonction de la solubilité de la lignine non-phénolique de départ, de la température de la réaction et/ou de la viscosité du mélange réactionnel.

Etape de dépolymérisation

Les inventeurs ont découvert qu'en mettant en présence la lignine non-phénolique oxydée issue de la première étape du procédé de l'invention avec un agent oxydant, on parvenait à dépolymériser efficacement la structure de la lignine pour obtenir des produits de dépolymérisation de la lignine.

Par « agent oxydant », on entend une substance apte à oxyder les espèces présentes dans le milieu réactionnel. De préférence, l'agent oxydant est nucléophile.

A titre d'agent oxydant nucléophile, on peut citer par exemple les peroxydes, comme le peroxyde d'hydrogène ($H_2O_2$) et le peroxyde de benzoyle, ou bien des composés tels que $O_3$, $KMnO_4$ et $NaIO_4$, ou encore tout oxydant nucléophile classiquement utilisé en chimie organique.

L'agent oxydant est de préférence le peroxyde d'hydrogène ($H_2O_2$).

Le peroxyde d'hydrogène est typiquement disponible sous forme d'une solution aqueuse à 35% massique, ce qui équivaut à une concentration molaire d'environ 10 M.

De préférence, typiquement lorsque l'agent oxydant est le peroxyde d'hydrogène, le rapport de la quantité d'agent oxydant mise en présence (en mol) sur la quantité de lignine non-phénolique oxydée présente dans le milieu réactionnel (en grammes) est compris entre 0,01/1 et 0,02/1.

La deuxième étape du procédé a lieu de préférence en milieu basique, notamment lorsque l'agent oxydant est le peroxyde d'hydrogène. Ainsi, lorsque la première étape du procédé a eu lieu en milieu de pH neutre ou acide, il est alors nécessaire de passer en milieu basique, et ce, de préférence avant de mettre en présence l'agent oxydant. Pour passer en milieu basique on peut utiliser toute méthode connue en soi, comme l'addition d'une une solution aqueuse basique, typiquement de soude, dans le milieu réactionnel.

Mise en oeuvre du procédé

Une mise en oeuvre typique du procédé de l'invention va maintenant être décrite.

Les étapes du procédé sont typiquement mises en oeuvre l'une à la suite de l'autre dans le même réacteur.

Alternativement, on peut effectuer ces deux étapes de manière séparée, dans deux réacteurs distincts.

Selon un mode de réalisation particulier, dans un réacteur muni d'un système de chauffage, d'un moyen d'agitation, et éventuellement d'un système réfrigérant, on introduit la lignine non-phénolique et une partie du solvant et on agite jusqu'à dissolution complète. On ajoute ensuite une solution de médiateur rédox dans une partie du solvant, puis on ajoute une solution de laccase dans le reste du solvant.

On met ensuite en présence la source d'oxygène, de préférence de manière continue, typiquement par bullage dans le milieu réactionnel lorsque la source d'oxygène est gazeuse.

La température du mélange est typiquement maintenue entre 20°C et 60°C, de préférence autour de 40°C,

jusqu'à ce qu'on atteigne l'oxydation totale de la lignine non-phénolique. A ce stade, la première étape du procédé est achevée.

On peut ensuite récupérer la lignine non-phénolique oxydée.

Alternativement, on peut avantageusement poursuivre le procédé en effectuant la deuxième étape dans le même réacteur. Selon ce mode, on peut se placer en pH basique en basifiant si nécessaire le milieu réactionnel.

On peut ensuite additionner l'agent oxydant, de préférence de manière continue, typiquement au goutte à goutte, lorsque l'agent oxydant est une solution.

La température du mélange est typiquement maintenue supérieure à 70°C, typiquement autour de 90°C.

L'avancement de la réaction est suivi par SEC (chromatographie par exclusion stérique).

On poursuit l'agitation et le chauffage du milieu réactionnel jusqu'à obtenir le degré de dépolymérisation souhaité.

Une fois la dépolymérisation terminée, les produits de dépolymérisation de la lignine peuvent être récupérés, en utilisant les techniques de purification classiques dans le domaine, à savoir la filtration, l'extraction, la distillation et/ou la séparation par chromatographie. Notamment, le solvant peut être retiré par évaporation, éventuellement sous pression réduite.

Le procédé de l'invention va maintenant être illustré au moyen d'exemples et d'exemples comparatifs.

EXEMPLES

<u>Réactifs</u>

Liqueur noire (fournie par SMURFIT Kappa)
Diméthylsulfate (commercialisé par Sigma Aldrich)
Lignine de bagasse de canne à sucre (fournie par SOLVAY) 2,2-azino-bis-(3-éthylbenzothiazoline-6-acide sulfonique) (ABTS) (commercialisé par Sigma Aldrich)
Laccase de *Trametes versicolor* (commercialisé par Sigma Aldrich)
Tampon acétate (pH = 4 ; 50 mM)
Peroxyde d'hydrogène (35%) (commercialisé par Sigma Aldrich)

<u>Exemple 1 - Extraction de la lignine Kraft à partir de la liqueur noire</u>

La lignine Kraft a été extraite de la liqueur de cuisson (commercialisée par SMURFIT KAPPA) par un traitement acide, de façon à la faire précipiter. Un post-traitement à l'éthanol permet d'éliminer les sels de sodium résiduels.

On dissout 800 g de liqueur noire dans 2 L d'eau distillée et le mélange est lentement acidifié (de pH = 13 à pH = 1,5) à l'aide d'une solution de HCl 6N. Le mélange est ensuite centrifugé à 4000 rpm pendant 10 minutes, le culot est récupéré et à nouveau traité avec une solution de HCl à pH = 1,5 et à nouveau centrifugé (refaire 3 fois avec HCl à pH = 1,3). On ajoute ensuite de l'éthanol au solide obtenu. Après filtration, la phase éthanol est évaporée et le résidu est lavé avec une solution de HCl à pH = 1,5 pour éliminer les sels résiduels. Après centrifugation, le culot est récupéré et lyophilisé. On obtient 180 g de lignine Kraft.

Des 180 g de lignine Kraft ainsi obtenus, on prélève 10 g que l'on dissout dans 200 mL de tétrahydrofurane (THF). Après agitation pendant 30 minutes sous ultrasons, le mélange est filtré pour éliminer le dépôt solide blanc, insoluble dans le THF. Après évaporation du THF, on obtient 9,2 g d'un solide brun. Ce solide brun est dissout dans 100 mL de diéthyléther ($Et_2O$). Une fraction reste insoluble dans l'$Et_2O$ (8,1 g) et la fraction soluble (0,9 g) est analysée par GC-MS après silylation afin de rendre volatils les composés.

Cette analyse met en évidence la présence de monophénols comme la vanilline ou l'acide vanillique et des résidus de la dégradation des sucres. La fraction insoluble est composée de lignine Kraft purifiée, qui peut être utilisée par la suite pour la méthylation et le procédé de dépolymérisation de la présente invention.

<u>Exemple 2A - Méthylation de la lignine Kraft</u>

On dissout 1 g de lignine Kraft dans 20 mL d'une solution de NaOH à 0,7 M (0,56 g) et le mélange est agité à température ambiante pendant 10 minutes, puis 0,8 mL de diméthylsulfate ($(MeO)_2SO_2$) sont lentement additionnés (gouttes à gouttes). L'agitation se poursuit pendant 30 minutes à température ambiante. Le mélange réactionnel est ensuite chauffé à 80 °C pendant 4 heures, en ajoutant une solution de NaOH à 0,7 M pour homogénéiser le milieu réactionnel (environ 10 mL de NaOH à 0,7 M supplémentaire sont ajoutés). Après 4 heures de réaction, le mélange est ramené à température ambiante et acidifié à l'aide d'une solution de HCl à 2 M jusqu'à pH = 2. Le brut réactionnel est filtré et le solide obtenu est lavé à l'eau distillée puis séché par lyophilisation. On obtient 0,874 g de lignine méthylée.

Exemple 2B - Méthylation de la lignine de bagasse de canne à sucre

La lignine de bagasse de canne à sucre a été fournie par SOLVAY a été utilisée sans prétraitement préalable.
La lignine de bagasse est traitée dans les conditions décrites à l'Exemple 2A. On obtient 0,92 g de lignine méthylée.

Exemple 3A - Dépolymérisation de lignine méthylée (issue de lignine Kraft)

On dissout 1 g de lignine méthylée obtenue à l'Exemple 2A dans 25 mL de dioxane et le mélange est agité à température ambiante jusqu'à dissolution complète.
Puis, on prépare une solution de 51 mg de 2,2-azino-bis-(3-éthylbenzothiazoline-6-acide sulfonique) (ABTS) (0,1 mmol) dans 1 mL de tampon acétate (pH = 4 ; 50 mM), que l'on ajoute à la solution de lignine méthylée.
On prépare ensuite une solution de 5 mg de laccase dans 200 mL de tampon acétate, on prélève 24 mL de cette solution que l'on additionne lentement au milieu contenant la lignine méthylée à 40 °C. Après addition de la laccase, de l'oxygène gazeux est introduit dans le mélange par bullage pendant 1 heure à 40 °C, en utilisant un ballon rempli d'oxygène.
Après 22 heures de réaction à 40 °C, on additionne 1 mL d'une solution de NaOH à 3 M, puis on additionne lentement 1 mL d'une solution aqueuse de peroxyde d'hydrogène (35 %, 10 mmol), le mélange étant agité à 90 °C.
L'avancement de la réaction est suivi par SEC (chromatographie par exclusion stérique). L'analyse par SEC est réalisée en utilisant trois colonnes de type TSK-gel (3000 PW, 4000 PW, 3000 PW) couplées en série, avec 1 M d'hydroxyde sodium jusqu'à pH = 12 et $NaN_3$ (3 %) dans de l'eau osmosée comme éluant. Le débit est de 1 mL/min et la détection se fait par UV à une longueur d'onde de 280 nm.
La première étape de la réaction (action du système laccase/ABTS) conduit à une faible dépolymérisation. La deuxième étape (action du peroxyde d'hydrogène) conduit à une forte dépolymérisation, avec la formation de molécules de faible masse moléculaire qui ont été isolées et identifiées.
Après 40 heures de réaction, le mélange est ramené à pH = 6-7 à l'aide d'une solution de HCl 1 N et le brut réactionnel est extrait avec du dichlorométhane (3x50 mL). Après séchage de la phase organique sur sulfate de sodium ($Na_2SO_4$) et évaporation des solvants volatils, on purifie le brut par chromatographie flash (avec comme éluant : dichlorométhane/méthanol 99/1 à 90/10).
On a ainsi isolé 30 mg d'acide 3,4-diméthoxybenzoïque à partir de 1 g de lignine méthylée obtenue à l'Exemple 2A.

Exemple comparatif 1

A titre de comparaison, on a soumis la lignine Kraft purifiée obtenue à l'Exemple 1 à l'action du système laccase/ABTS, en appliquant directement les conditions réactionnelles décrites à l'Exemple 3A (c'est-à-dire sans effectuer de méthylation de la lignine).
L'avancement de la réaction est suivi par SEC (conditions décrites ci-dessus) :

| t (heures) | $M_p$ | $M_n$ | $M_w$ |
|---|---|---|---|
| 0 | 661 | 902 | 1326 |
| 2 | 842 | 1254 | 1662 |
| 73 | 905 | 1036 | 1821 |

$M_p$ : pic de poids moléculaire, en g/mol
$M_n$ : masse moléculaire moyenne en nombre, en g/mol
$M_w$ : masse moléculaire moyenne en masse, en g/mol

On observe que la masse moléculaire moyenne des espèces en présence augmente avec la durée de réaction.
Ainsi, lorsque l'action du système laccase/ABTS est réalisée sur de la lignine Kraft non-méthylée, on n'observe pas de dépolymérisation, mais au contraire une augmentation de la masse moléculaire de la lignine.

Exemple 3B - Dépolymérisation de lignine méthylée (issue de lignine de bagasse)

On a traité la lignine méthylée obtenue à l'Exemple 2B en appliquant les conditions réactionnelles décrites à l'Exemple 3A (l'action de la laccase est prolongée pendant 65 heures, après quoi on additionne le peroxyde d'hydrogène).

L'avancement de la réaction est suivi par SEC (conditions décrites ci-dessus) :

| t (heures) | $M_p$ | $M_n$ | $M_w$ |
|---|---|---|---|
| 0 | 5054 | 1414 | 10245 |
| 2 | 4806 | 759 | 8681 |
| 65 | 3555 | 269 | 6857 |
| 70 | 2443 | 1601 | 4907 |
| 80 | 1525 | 130 | 2112 |

La première étape de la réaction (laccase/ABTS) conduit à une faible dépolymérisation. La deuxième étape (addition du peroxyde d'hydrogène à t = 65 h) conduit à la dépolymérisation avec la production de phénols de faible masse moléculaire qui n'ont pas été isolés et identifiés.

Exemple comparatif 2

A titre de comparaison, on a soumis la lignine de bagasse à l'action du système laccase/ABTS, en appliquant directement les conditions réactionnelles décrites à l'Exemple 3A (c'est-à-dire sans effectuer de méthylation de la lignine).
L'avancement de la réaction est suivi par SEC (conditions décrites ci-dessus) :

| t (heures) | $M_p$ | $M_n$ | $M_w$ |
|---|---|---|---|
| 0 | 2254 | 145 | 4957 |
| 1 | 4048 | 417 | 8245 |
| 24 | 9242 | 4282 | 17329 |

On observe que la masse moléculaire moyenne des espèces en présence augmente avec la durée de réaction.
Ainsi, lorsque l'action du système laccase/ABTS est réalisée sur de la lignine de bagasse non-méthylée, on n'observe pas de dépolymérisation, mais au contraire une augmentation de la masse moléculaire de la lignine.

Exemple comparatif 3

A titre de comparaison, on a soumis la lignine méthylée obtenue à l'Exemple 2B à l'action du peroxyde d'hydrogène en milieu basique, sans la soumettre à l'action du système laccase/ABTS auparavant.
On dissout 1 g de lignine méthylée obtenue à l'Exemple 2B dans 25 mL de dioxane et le mélange est agité à température ambiante jusqu'à dissolution complète.
Puis, 25 mL d'eau osmosée sont ajoutés au mélange.
On additionne 1 mL d'une solution de NaOH à 3 M, puis on additionne lentement 1 mL d'une solution aqueuse de peroxyde d'hydrogène (35 %, 10 mmol), le mélange étant agité à 90 °C.
L'avancement de la réaction est suivi par SEC (conditions décrites ci-dessus) :

| t (heures) | $M_p$ | $M_n$ | $M_w$ |
|---|---|---|---|
| 0 | 5054 | 1414 | 10245 |
| 5 | 2541 | 404 | 4651 |
| 15 | 2394 | 153 | 4725 |

On observe une dépolymérisation de la lignine méthylée moins importante que lorsqu'elle est traitée au préalable par l'action du système laccase/ABTS.
Sans vouloir être lié à une théorie particulière, cette dépolymérisation partielle s'explique par le fait que, dans la lignine (et donc dans la lignine méthylée), un certain pourcentage de fonctions hydroxyles destinées à être oxydées par la mise en présence du système laccase/médiateur sont déjà à l'état oxydé, c'est-à-dire sous forme de cétones. La rupture oxydante des liaisons C-C au voisinage de ces fonctions cétones provoque une dépolymérisation partielle.

Cet exemple montre néanmoins que la première étape du procédé est nécessaire pour optimiser l'action dépolymérisante du peroxyde d'hydrogène.

L'analyse comparative des lignines de bagasse obtenues à l'issue des Exemples 2B, 3B et à l'issue de l'Exemple comparatif 3 met en évidence que la fragmentation de la lignine est la plus efficace lorsque l'on soumet successivement la lignine méthylée à l'action du système laccase/ABTS puis à l'action du peroxyde d'hydrogène :

| | |
|---|---|
| Lignine de l'Exemple 2B, non dépolymérisée | $M_{lig}$ = 10245 |
| Lignine de l'Exemple 3B, traitée avec laccase+$H_2O_2$ | $M_w$ = 2112 |
| Lignine de l'Exemple comparatif 3, traitée avec $H_2O_2$ | $M_w$ = 4725 |

## Revendications

1. Procédé de dépolymérisation de la lignine, comprenant :

   - une étape d'oxydation de lignine non-phénolique par mise en présence, dans au moins un solvant, de lignine non-phénolique, de laccase, d'un médiateur rédox choisi dans le groupe constitué du 2,2-azino-bis-(3-éthylbenzothiazoline-6-acide sulfonique) (ABTS), de l'acide violurique, de l'acide hydroxyanthranilique, du TEMPO et du N-hydroxyphtelimide, et d'une source d'oxygène, ce par quoi on obtient un mélange comprenant de la lignine non-phénolique oxydée, et
   - une étape de dépolymérisation de la lignine non-phénolique oxydée ainsi obtenue, par addition d'un agent oxydant nucléophile,

   ladite lignine non-phénolique étant de la lignine alkylée obtenue à partir de lignine phénolique par fonctionnalisation de fonctions phénol de ladite lignine phénolique par des alkyles, pour donner des fonctions alcoxy.

2. Procédé selon la revendication 1, dans lequel la lignine non-phénolique est de la lignine méthylée.

3. Procédé selon la revendication 2, dans lequel la lignine méthylée est obtenue par mise en présence, dans une solution aqueuse basique, de lignine phénolique et d'un agent méthylant.

4. Procédé selon la revendication 3, dans lequel l'agent méthylant est le diméthylsulfate.

5. Procédé selon l'une quelconque des revendications 1 à 4, dans lequel la source d'oxygène est l'oxygène pur ou l'air.

6. Procédé selon l'une quelconque des revendications 1 à 5, dans lequel le médiateur rédox est le 2,2-azino-bis-(3-éthylbenzothiazoline-6-acide sulfonique).

7. Procédé selon l'une quelconque des revendications 1 à 6, dans lequel l'agent oxydant est le peroxyde d'hydrogène.

8. Procédé selon l'une quelconque des revendications 1 à 7, dans lequel l'étape d'oxydation est réalisée en milieu basique.

9. Procédé selon l'une quelconque des revendications 1 à 8, dans lequel la lignine non-phénolique est issue de la fonctionnalisation de fonctions phénol de lignine Kraft ou de la lignine de bagasse de canne à sucre.

## Patentansprüche

1. Verfahren zur Depolymerisation von Lignin, umfassend:

   - einen Schritt der Oxidation von nicht-phenolischem Lignin durch Einbringen von nicht-phenolischem Lignin, Laccase, einem Redoxmediator ausgewählt aus der Gruppe bestehend aus 2,2-Azino-di(3-ethylbenzthiazolin-6-sulfonsäure) (ABTS), Violursäure, Hydroxyanthranilsäure, TEMPO und N-Hydroxyphtalimid, und einer Sauerstoffquelle in mindestens ein Lösemittel, wodurch eine Mischung umfassend das oxidierte Lignin erhalten wird, und

- einen Schritt der Depolymerisation des so erhaltenen oxidierten Lignins durch Zugabe eines nukleophilen Oxidationsmittels,
wobei das nicht-phenolische Lignin alkyliertes Lignin ist, erhalten ausgehend von phenolischem Lignin durch Funktionalisierung der Phenolfunktionen des phenolischem Lignins durch Alkyle, um Alkoxyfunktionen zu erhalten.

**2.** Verfahren gemäß Anspruch 1, wobei das nicht-phenolische Lignin methyliertes Lignin ist.

**3.** Verfahren gemäß Anspruch 2, wobei das methylierte Lignin erhalten wird durch Einbringen von phenolischem Lignin und einem Methylierungsmittel in eine basische wässrige Lösung.

**4.** Verfahren gemäß Anspruch 3, wobei das Methylierungsmittel Dimethylsulfat ist.

**5.** Verfahren gemäß einem der Ansprüche 1 bis 4, wobei die Sauerstoffquelle reiner Sauerstoff oder Luft ist.

**6.** Verfahren gemäß einem der Ansprüche 1 bis 5, wobei der Redoxmediator 2,2-Azino-di(3-ethylbenzthiazolin-6-sulfonsäure) ist.

**7.** Verfahren gemäß einem der Ansprüche 1 bis 6, wobei das Oxidationsmittel Wasserstoffperoxid ist.

**8.** Verfahren gemäß einem der Ansprüche 1 bis 7, wobei der Schritt der Oxidation in basischem Milieu durchgeführt wird.

**9.** Verfahren gemäß einem der Ansprüche 1 bis 8, wobei das nicht-phenolische Lignin von der Funktionalisierung der Phenolfunktionen des Kraft-Lignins oder des Lignins von Zuckerrohr-Bagasse stammt.

## Claims

**1.** A method for depolymerization of lignin, comprising:

- a step for oxidization of non-phenolic lignin by putting into presence, in at least one solvent, non-phenolic lignin, laccase, a redox mediator selected from the group consisting of 2,2-azino-bis-(3-ethylbenzothiazoline-6-sulfonic acid) (ABTS), violuric acid, hydroxyanthranilic acid, TEMPO, and N-hydroxyphthalimide, and an oxygen source, whereby a mixture comprising oxidized non-phenolic lignin is obtained, and
- a step for depolymerization of the oxidized non-phenolic lignin being thereby obtained, by adding of a nucleophilic oxidizer,
said non-phenolic lignin being alkylated lignin obtained from phenolic lignin by functionalization of phenol functions of said phenolic lignin by alkyls, for giving alkoxy functions.

**2.** The method according to claim 1, wherein the non-phenolic lignin is methylated lignin.

**3.** The method according to claim 2, wherein the methylated lignin is obtained by putting into presence, in a basic aqueous solution, phenolic lignin and a methylation agent.

**4.** The method according to claim 3, wherein the methylation agent is dimethyl sulfate.

**5.** The method according to any one of claims 1 to 4, wherein the oxygen source is pure oxygen or air.

**6.** The method according to any one of claims 1 to 5, wherein the redox mediator is 2,2-azino-bis-(3-ethylbenzothiazoline-6-sulfonic acid).

**7.** The method according to any one of claims 1 to 6, wherein the oxidizer is hydrogen peroxide.

**8.** The method according to any one of claims 1 to 7, wherein the oxidation step is carried out in a basic medium.

**9.** The method according to any one of claims 1 to 8, wherein the non-phenolic lignin stems from the functionalization of phenol functions of a Kraft lignin or sugarcane bagasse lignin.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2013090430 A

### Littérature non-brevet citée dans la description


- **SREBOTNIK, E. et al.** Dégradation of nonphenolic lignin by the laccase/1-hydroxybenzotriazole system. *Journal of Biotechnology,* 25 Août 2000, vol. 81 (2-3), 179-188
- **BOURBONNAIS, R. et al.** Lignin oxidation by laccase isozymes from Trametes versicolor and role of the mediator 2,2'-azinobis(3-ethylbenzthiazoline-6-sulfonate) in kraft lignin depolymerization. *Applied and Environmental Microbiology,* 01 Mai 1995, vol. 61 (5), 1876-1880
- **KAPICH, A. et al.** Involvement of lipid peroxidation in the dégradation of a non-phenolic lignin model compound by manganese peroxidase of the litter-decomposing fungus Stropharia coronilla. *Biochemical and Biophysical Research Communications,* 06 Mai 2005, vol. 330 (2), 371-377
- **PATRICK J COLLINS ; ALAN D W DOBSON ; JIM A FIELD.** Réduction of the 2,2'-Azinobis(3-Ethylbenzthiazoline-6-Sulfonate) Cation Radical by Physiological Organic Acids in the Absence and Presence of Manganese. *Applied and Environmental Microbiology,* Juin 1998, vol. 64 (6), 2026-2031
- **SREBOTNIK et al.** *Journal of Biotechnology,* 2000, vol. 81, 179-188
- **GRANAT et al.** *J. Agric. Food Chem.,* 1995, vol. 43 (6), 1538-1544
- **SADEGHIFAR et al.** *Ind. Eng. Chem. Res.,* 2012, vol. 51, 16713-16720
- **LIGNINS ; LIGNANS.** Advances in Chemistry. Taylor&Francis Group, 2010
- **BOURBONNAIS R.** *Appl. Enviro. Microbiol.,* 1995, vol. 61, 1876-1880
- **CHAKAR et al.** *Can. J. Chem.,* 2004, vol. 82, 344-352